(19) Europäisches Patentamt
European Patent Office
Office européen des brevets

(11) EP 3 604 352 B1

(12) **EUROPEAN PATENT SPECIFICATION**

(45) Date of publication and mention of the grant of the patent:
**19.02.2025 Bulletin 2025/08**

(21) Application number: **18774691.2**

(22) Date of filing: **15.03.2018**

(51) International Patent Classification (IPC):
***C08F 2/38*** *(2006.01)* ***C08F 20/06*** *(2006.01)*
***C07C 69/00*** *(2006.01)*

(52) Cooperative Patent Classification (CPC):
**C07C 53/19; C07C 69/675; C07C 233/05; C08F 2/38; C08F 120/06; C08F 120/34**

(86) International application number:
**PCT/JP2018/010242**

(87) International publication number:
**WO 2018/180547 (04.10.2018 Gazette 2018/40)**

(54) **METHOD FOR PRODUCING A POLYMER USING A MOLECULAR-WEIGHT CONTROLLING AGENT FOR RADICAL POLYMERIZATION**

VERFAHREN ZUR HERSTELLUNG EINES POLYMERS UNTER VERWENDUNG EINES MOLEKULARGEWICHTSSTEUERNDEN MITTELS FÜR RADIKALISCHE POLYMERISATION

PROCÉDÉ DE PRODUCTION DE POLYMÈRE UTILISANT UN AGENT DE RÉGULATION DE POIDS MOLÉCULAIRE POUR POLYMÉRISATION RADICALAIRE,

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2017 JP 2017067513**

(43) Date of publication of application:
**05.02.2020 Bulletin 2020/06**

(73) Proprietor: **Godo Shigen Co., Ltd.**
**Chiba, 299-4333 (JP)**

(72) Inventors:
- **MIYAJIMA Toru**
  **Tokyo 103-0023 (JP)**
- **MATSUBARA Yusuke**
  **Tokyo 103-0023 (JP)**
- **MIYAMOTO Michihiko**
  **Chiba 299-4333 (JP)**
- **KOMATSU Hiroto**
  **Chiba 299-4333 (JP)**
- **YAMAGUCHI Yu**
  **Chiba 299-4333 (JP)**
- **JITSUKAWA Takuya**
  **Chiba 299-4333 (JP)**

(74) Representative: **Vossius & Partner**
**Patentanwälte Rechtsanwälte mbB**
**Siebertstrasse 3**
**81675 München (DE)**

(56) References cited:
**WO-A1-2017/057706** **FR-A1- 2 892 120**
**JP-A- 2000 143 728** **JP-A- 2000 502 144**
**JP-A- 2001 520 282** **JP-A- 2007 308 665**
**JP-A- 2011 079 979** **JP-A- 2012 062 449**
**JP-A- 2013 060 501** **JP-A- H11 140 127**
**JP-A- S5 744 609** **JP-A- S6 264 814**
**US-A- 2 916 470** **US-A1- 2009 082 224**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 604 352 B1

## Description

TECHNICAL FIELD

The present invention relates to a method for producing a polymer using a molecular weight controlling agent for radical polymerization, and a polymer produced using the method.

BACKGROUND ART

Heretofore, from the viewpoint of promoting development of innovative functional materials, polymerization of vinyl-based monomers utilizing high reactivity of radicals has been carried out by various production methods. As a method of polymerizing a vinyl monomer to obtain a vinyl polymer, a radical polymerization method is known. The radical polymerization method is widely used in various industrial fields as a simple and highly versatile method, but it has a disadvantage that molecular weight control is difficult and a resulting vinyl polymer tends to be a mixture of polymers having various molecular weights.

As a method of solving such a defect, a controlled radical polymerization method has been developed since circa 1990. According to the controlled radical polymerization method, it is possible to control molecular weight, and it is possible to obtain a polymer having a narrower molecular weight distribution as compared with conventional radical polymerization methods. For this reason, controlled radical polymerization can exhibit superior properties of controlled polymerization as well as those of radical polymerization, and is in the spotlight as a method for producing high quality and highly functional polymers.

As specific methods of the controlled radical polymerization method, a method using an initiator composed of a specific transition metal complex, an organic halide and a Lewis acid (Patent Document 1), a method using a thiocarbonyl compound as a chain transfer agent (Patent Document 2), a method of performing polymerization in the presence of a specific organic tellurium compound (Patent Document 3), etc. are known.

Patent Document 4 discloses a method for producing an acrylic polymer wherein the radical polymerisation of (meth)acrylic acid esters having alkyl groups of 1-12 carbons is progressed in the presence of an iodine compound having at least one functional group in the molecule, and stopping the radical polymerisation by using a chain transfer agent having a functional group in the molecule.

Patent Document 5 discloses a method for producing water-absorbent resin particles, comprising a step of polymerizing a monomer composition comprising a water-soluble vinyl monomer (a1) and/or a vinyl monomer (a2) that turns into a water-soluble vinyl monomer (a1) through hydrolysis and a crosslinking agent (b) in the presence of at least one organic main group element compound selected from the group consisting of organic iodine compounds, organic tellurium compounds, organic antimony compounds, and organic bismuth compounds.

Patent Document 6 discloses a method for producing copolymers characterised in that copolymerisation is carried out between a macromonomer (A), which has been emulsified aqueously in advance, and a vinyl compound and/or a conjugated diene compound (B), or an aqueous emulsion dispersion of the compound (B), using a radical initiator.

Patent Document 7 discloses a living radical polymerisation method, comprising the process of polymerising a reaction liquid containing a radical-reactive monomer, a radical initiator and an organohalide having a carbon-halogen bond in a reaction vessel, wherein in the polymerisation process, per ml of volume of the liquid phase in the reaction vessel of oxygen in the gas phase in the reaction vessel is 1 to 70 millimoles of oxygen in the gas phase in the reaction vessel.

PATENT DOCUMENTS

Patent Document 1: JP-A-11-158206
Patent Document 2: JP-A-2000-515181
Patent Document 3: JP-A-2006-299278
Patent Document 4: JP-H11-140127 A
Patent Document 5: WO 2017/057706 A1
Patent Document 6: JP-S62-64814 A
Patent Document 7: JP-2012-062449 A

## SUMMARY OF THE INVENTION

### PROBLEMS TO BE SOLVED BY THE INVENTION

The methods described in Patent Documents 1 to 3, however, are difficult to apply to a polymerization reaction in an aqueous medium because the compounds to be used do not have solubility in water, and thus are difficult to apply to polymerization of water-soluble monomers. From the viewpoint of environmental load reduction and economy, development of a controlled radical polymerization method applicable to an aqueous system is desired under increasing industrial importance of a method of polymer production involving carrying out radical polymerization of a water-soluble monomer in an aqueous medium. An object of the present invention is to provide a molecular weight controlling agent for radical polymerization which enables a method of controlled radical polymerization of water-soluble monomers in an aqueous medium, a method for producing a polymer of a water-soluble vinyl monomer using the same, and a polymer of a water-soluble vinyl monomer.

### SOLUTIONS TO THE PROBLEMS

The present disclosure provides a molecular weight controlling agent for radical polymerization containing an iodine compound represented by the following formula (1) as an active ingredient, in which a solubility of the active ingredient in water is 0.5% by weight or more at 20°C.

[Chemical Formula 1]

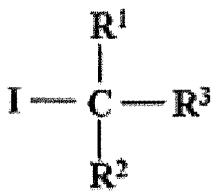
(1)

In the formula, $R^1$ is -COOX, -$CONR^4R^5$, an aromatic group or a cyano group, X is a hydrogen atom, an aliphatic group, an alkali metal, an alkaline earth metal, an organic ammonium or an ammonium, and $R^2$, $R^3$, $R^4$ and $R^5$ each independently represent a hydrogen atom, an aromatic group or an aliphatic group.

The present invention provides a method for producing a polymer, including a step of carrying out radical polymerization of a raw material vinyl monomer containing a water-soluble vinyl monomer (a1), which is soluble in an amount of at least 100 g in 100 g of water at 25 °C, and/or a vinyl monomer (a2) that becomes the water-soluble vinyl monomer (a1) through hydrolysis, in which the radical polymerization is carried out in the presence of water and the above-mentioned molecular weight controlling agent for radical polymerization, and is performed by aqueous solution polymerization or inverse phase suspension polymerization.

Furthermore, the present invention is also a polymer to be produced by the above-mentioned production method.

### ADVANTAGES OF THE INVENTION

The molecular weight controlling agent for radical polymerization of the present invention exerts a superior molecular weight control effect in an aqueous medium. Moreover, by the production method of the present invention, a polymer of a water-soluble monomer with a narrow molecular weight distribution can be produced simply by using the molecular weight controlling agent for radical polymerization.

### BRIEF DESCRIPTION OF THE DRAWINGS

Fig. 1 is a drawing schematically illustrating a cross-sectional view of a filtration cylinder for measuring a gel liquid permeation rate.

Fig. 2 is a perspective view schematically illustrating a pressing axis and weights for measuring a gel liquid permeation rate.

MODE FOR CARRYING OUT THE INVENTION

Hereafter, a method for producing a polymer using a molecular weight controlling agent for radical polymerization according to embodiments of the present invention are described in detail.

The molecular weight controlling agent for radical polymerization of the present invention comprises an iodine compound represented by the above formula (1) as an active ingredient, and a solubility of the active ingredient in water exhibits 0.5% by weight or more at 20°C.

In the above chemical formula (1), $R^1$ is -COOX, - $CONR^4R^5$, an aromatic group or a cyano group, X is a hydrogen atom, an aliphatic group, an alkali metal, an alkaline earth metal, an organic ammonium or an ammonium, and $R^2$, $R^3$, $R^4$ and $R^5$ each independently represent a hydrogen atom, an aromatic group or an aliphatic group.

The aliphatic group as $R^2$, $R^3$, $R^4$, $R^5$ and X includes an optionally substituted, linear or branched aliphatic hydrocarbon group having 1 to 12 carbon atoms or an alicyclic hydrocarbon group having 3 to 12 carbon atoms, and preferably is a linear or branched alkyl group having 1 to 12 carbon atoms. When the aliphatic group is substituted, the number of substituents is not particularly limited as long as the group can be substituted, and is 1, or 2 or more.

Examples of the linear or branched aliphatic hydrocarbon group having 1 to 12 carbon atoms include linear alkyl groups (e.g., methyl, ethyl, n-propyl, n-butyl, n-octyl and n-dodecyl groups) and branched alkyl groups (e.g., isopropyl, isobutyl, sec-butyl, tert-butyl and 2-ethylhexyl groups).

Examples of the alicyclic hydrocarbon group having 3 to 12 carbon atoms include a cyclopropyl group, a cyclobutyl group, a cyclopentyl group, a cyclohexyl group, a bicyclohexyl group, a cyclooctyl group, a cyclohexylmethyl group, a cyclohexylethyl group and a methylcyclohexyl group.

Further, examples of the group which may substitute on the aliphatic group include a halogen atom, a hydroxyl group, an optionally substituted, linear or branched alkyl group having 1 to 12 carbon atoms, an optionally substituted aromatic group, an optionally substituted non-aromatic heterocyclic group, a linear or branched alkoxy group having 1 to 12 carbon atoms, a cyano group, a carboxyl group, or a nitro group.

Examples of the alkali metal, alkaline earth metal and organic ammonium in X include alkali metals: sodium, potassium, etc., alkaline earth metals: calcium, magnesium, etc., and organic ammonium: trimethylammonium, tetramethylammonium, triethylammonium, ethyltrimethylammonium, tetraethyl ammonium, etc.

Examples of the aromatic group in $R^1$, $R^2$, $R^3$, $R^4$ and $R^5$ include an aromatic hydrocarbon ring group or an aromatic heterocyclic group, and more specifically include a phenyl group, a biphenylyl group, a terphenylyl group, a naphthyl group, a binaphthylyl group, an azulenyl group, an anthracenyl group, a phenanthrenyl group, a furyl group, a thienyl group, a pyrrolyl group, a pyrazolyl group, an imidazolyl group, an isoxazolyl group, a thiazolyl group, a thiadiazolyl group, a pyridyl group, a benzofuranyl group, an indolyl group, a benzothiazolyl group, and a carbazolyl group.

The aromatic group may be substituted, and the number of substituents in this case is not particularly limited as long as they are substitutable, and is 1, or 2 or more.

Further, examples of the group which may substitute on the aromatic group include a halogen atom, a hydroxyl group, an optionally substituted, linear or branched alkyl group having 1 to 12 carbon atoms, an optionally substituted aromatic group, an optionally substituted non-aromatic heterocyclic group, a carboxyl group, a linear or branched alkoxy group having 1 to 12 carbon atoms, a cyano group, or a nitro group.

Examples of the linear or branched alkyl group having 1 to 12 carbon atoms specifically include a methyl group, an ethyl group, a n-propyl group, an isopropyl group, a n-butyl group, an isobutyl group, a sec-butyl group, a tert-butyl group, a cyclopropyl group, a cyclobutyl group, a cyclopentyl group, and a cyclohexyl group.

Examples of the linear or branched alkoxy group having 1 to 12 carbon atoms specifically include a methoxy group, an ethoxy group, a propoxy group, a butoxy group, an isopropoxy group, an isobutoxy group, a tert-butoxy group, a sec-butoxy group, an isopentyloxy group, a neopentyloxy group, a tert-pentyloxy group, and a 1,2-dimethylpropoxy group.

Examples of the halogen atom specifically include such atoms as a fluorine atom, a chlorine atom, a bromine atom, and an iodine atom.

The non-aromatic heterocyclic group refers to a monocyclic, bicyclic or tricyclic, 5- to 14-membered non-aromatic heterocyclic group containing one or more hetero atoms selected from the group consisting of a nitrogen atom, a sulfur atom, and an oxygen atom. Specific examples of the group include a pyrrolidinyl group, a pyrrolyl group, a piperidinyl group, a piperazinyl group, an imidazolyl group, a pyrazolidyl group, an imidazolidyl group, a morpholinyl group, a tetrahydrofuryl group, a tetrahydropyranyl group, a pyrrolinyl group, a dihydrofuryl group, a dihydropyranyl group, an imidazolinyl group, and an oxazolinyl group.

In the iodine compound represented by the formula (1), two or more groups of $R^2$, $R^3$, $R^4$, $R^5$, and X may be bonded to each other and form a saturated or unsaturated, 5- or 6-membered ring. Such examples include:

(i) rings in which $R^2$ and $R^3$ are bonded: a cyclopentane ring, a pyrrolidine ring, an oxazolidine ring, a thiazolidine ring, a tetrahydrofuran ring, a cyclohexane ring, a dioxane ring, a tetrahydrothiophene ring, etc.;
(ii) rings in which $R^4$ and $R^5$ are bonded: a pyrrolidine ring, a pyrrole ring, a triazole ring, piperidine ring, a piperazine

ring, a piperazinone ring, a morpholine ring, etc.;
(iii) rings in which X and $R^2$ or $R^3$ are bonded: a $\gamma$-butyrolactone ring, a $\delta$-valerolactone ring;
(iiii) rings in which $R^2$ and $R^4$ are bonded: a 2-pyrrolidone ring, a succinimide ring, a 2-piperidone ring, a glutarimide ring, etc.

Among the iodine compounds represented by the general formula (1), iodine compounds having a solubility in water of 0.5% by weight or more at 20°C include those having a hydrophilic functional group (specifically, a carboxyl group and a salt thereof, a hydroxyl group, an amide groups, etc.), and may further have a group that does not inhibit hydrophilicity. Specifically, at least one of $R^1$ to $R^3$ has a hydrophilic functional group or has a hydrophilic functional group as a substituent, and at least one of $R^1$ to $R^3$ may have a group that does not inhibit hydrophilicity while having a hydrophilic functional group or having no hydrophilic functional group. Examples of such compounds include 2-iodoacetic acid, 2-iodopropionic acid, 2-iodopropionitrile, 2-iodopropionic acid amide, 2-iodo-2-methylpropionic acid, 2-iodo-2-methylpropionic acid amide, sodium 2-iodo-2-methylpropionate, calcium 2-iodo-2-methylpropionate, ammonium 2-iodo-2-methylpropionate, 2-hydroxyethyl 2-iodo-2-methylpropionate, 2-iodopentanoic acid, 2,5-diiodoadipic acid, $\alpha$-iodo-$\gamma$-butyrolactone, sodium 2-iodo-2-phenylacetate, calcium 2-iodo-2-phenylacetate, ammonium 2-iodo-2-phenylacetate, and 2-hydroxyethyl 2-iodo-2-phenylacetate.

The molecular weight controlling agent for radical polymerization of the present invention comprises a water-soluble iodine compound represented by the formula (1) as an active ingredient. The above iodine compounds may be used singly or two or more species thereof may be used in combination. The molecular weight controlling agent for radical polymerization of the present invention may use the above-mentioned iodine compound as it is, and may take the form of liquid, powder, solid or the like as required. Moreover, it may take the form of an aqueous solution, encapsulation, etc. as necessary. In addition, various additives such as stabilizers and dispersing agents may be incorporated as necessary. Among these forms, it is preferable to take a liquid or powdery form from the viewpoint of handling, and more preferable to take an aqueous solution form.

The molecular weight controlling agent for radical polymerization of the present invention can be suitably used for radical polymerization of a water-soluble vinyl monomer. The molecular weight controlling agent for radical polymerization of the present invention exhibits a solubility of 0.5% by weight or more at 20°C in water with respect to the active ingredient thereof, and from the viewpoint of handling and molecular weight control at the time of radical polymerization, it is preferably dissolved completely during a step of carrying out radical polymerization of a water-soluble vinyl monomer, such as a water-soluble vinyl monomer (a1) and/or a vinyl monomer (a2) that becomes the water-soluble vinyl monomer (a1) through hydrolysis, in the presence of water.

The molecular weight controlling agent for radical polymerization of the present invention preferably has a solubility of 1% by weight or more at 20°C in water with respect to the active ingredient thereof, and in mixing with an aqueous monomer solution to be used for the present production method, the molecular weight controlling agent for radical polymerization is mixed instantaneously and homogeneously without causing phase separation from the aqueous monomer solution, and it is expected to stably exhibit a superior molecular weight control effect during the polymerization of a vinyl monomer.

Such molecular weight controlling agents for radical polymerization may be used singly or two or more species thereof may be used in combination.

The method for producing a polymer of the present invention is a method for producing a polymer, comprising a step of carrying out radical polymerization of a raw material vinyl monomer comprising a water-soluble vinyl monomer (a1) and/or a water-soluble vinyl monomer (a2) that becomes the water-soluble vinyl monomer (a1) through hydrolysis (hereinafter also referred simply to as hydrolyzable vinyl monomer (a2)), and the radical polymerization is carried out in the presence of the above-described molecular weight controlling agent for radical polymerization and water.

The water-soluble vinyl monomer (a1) is a vinyl monomer soluble in an amount of at least 100 g in 100 g of water at 25°C. The radical polymerization of the raw material vinyl monomer is performed by carrying out aqueous solution polymerization or inverse phase suspension polymerization.

In the production method of the present invention, the molecular weight controlling agent for radical polymerization has a molecular weight control effect that is enhanced as the solubility in water of the active ingredient increases. Although the mechanism of the action thereof is unknown, it is presumed that when the agent is uniformly mixed at the start of polymerization and the agent is high in affinity with a water-soluble vinyl monomer, an initiation reaction uniformly starts and the agent acts as dormant species during radical polymerization, and as a result, the molecular weight distribution of a resulting polymer can be narrowly controlled.

In the production method of the present invention, the molecular weight controlling agent for radical polymerization is preferably added as an aqueous solution from the viewpoint of molecular weight control, and it is more preferably added as an aqueous solution having a concentration of 0.5% by weight to 20% by weight in terms of the amount of active ingredients. In addition, also when using in another form, the usage can be set suitably with reference to the above-described usage.

The water-soluble vinyl monomer (a1) as used in the production method of the present invention is not particularly limited, and there can be used such conventional monomers as vinyl monomers having at least one water-soluble substituent and an ethylenically unsaturated group disclosed in paragraphs 0007 to 0023 of Japanese Patent No. 3648553 (e.g., anionic vinyl monomers, nonionic vinyl monomers, and cationic vinyl monomers), the anionic vinyl monomers, nonionic vinyl monomers, and cationic vinyl monomers disclosed in paragraphs 0009 to 0024 of JP-A-2003-165883, and vinyl monomers having at least one group selected from the group consisting of a carboxyl group, a sulfo group, a phosphono group, a hydroxyl group, a carbamoyl group, an amino group, and an ammonio group disclosed in paragraphs 0041 to 0051 of JP-A-2005-75982.

The hydrolyzable vinyl monomer (a2) is not particularly limited, and there can be used such conventional vinyl monomers as vinyl monomers having at least one hydrolyzable substituent that turns into a water-soluble substituent through hydrolysis disclosed in paragraphs 0024 to 0025 of Japanese Patent No. 3648553, and vinyl monomers having at least one hydrolyzable substituent [e.g., a 1,3-oxo-2-oxapropylene (-CO-O-CO-) group, an acyl group, and a cyano group] disclosed in paragraphs 0052 to 0055 of JP-A-2005-75982. The water-soluble vinyl monomer as used herein means a vinyl monomer soluble in an amount of at least 100 g in 100 g of water at 25°C. The hydrolyzability of the hydrolyzable vinyl monomer (a2) means a property to be hydrolyzed by the action of water and, according to need, of a catalyst (e.g., an acid or a base), thereby becoming water-soluble. Although the hydrolysis of the hydrolyzable vinyl monomer (a2) may be carried out during polymerization, after polymerization, or both during and after polymerization, after polymerization is preferred from the viewpoint of the solubility in water or the molecular weight or molecular weight distribution control of a resulting polymer.

Among these, preferred from the viewpoint of the solubility in water or the molecular weight or molecular weight distribution control of a resulting polymer are water-soluble vinyl monomers (a1), more preferred are anionic vinyl monomers and vinyl monomers having a carboxyl (salt) group, a sulfo (salt) group, an amino group, a carbamoyl group, an ammonio group, or a mono-, di- or tri-alkylammonio group, even more preferred are vinyl monomers having a carboxyl (salt) group or a carbamoyl group, particularly preferred are (meth)acrylic acid (salts) and (meth)acrylamide, particularly preferred are (meth)acrylic acid (salts), and most preferred are acrylic acid (salts).

The "carboxyl (salt) group" means a "carboxyl group" or a "carboxylate group", and the "sulfo (salt) group" means a "sulfo group" or a "sulfonate group". The (meth)acrylic acid (salt) means acrylic acid, a salt of acrylic acid, methacrylic acid, or a salt of methacrylic acid and the (meth)acrylamide means acrylamide or methacrylamide. Examples of such salts include salts of alkali metal (lithium, sodium, potassium, etc.), salts of alkaline earth metal (magnesium, calcium, etc.), and ammonium ($NH_4$) salts. Among these salts, salts of alkali metals and ammonium salts are preferred from the viewpoint of the solubility in water or the molecular weight or molecular weight distribution control of a polymer, more preferred are salts of alkali metals, and sodium salts are particularly preferred.

When one of a water-soluble vinyl monomer (a1) and a hydrolyzable vinyl monomer (a2) is contained as a raw material vinyl monomer, a single species of each of the monomers may be used singly or, alternatively, two or more species may be used as necessary. The same also applies to the case where both a water-soluble vinyl monomer (a1) and a hydrolyzable vinyl monomer (a2) are used. When both the water-soluble vinyl monomer (a1) and the hydrolyzable vinyl monomer (a2) are used, their contained molar ratio [(a1)/(a2)] is preferably from 75/25 to 99/1, more preferably from 85/15 to 95/5, particularly preferably from 90/10 to 93/7, and most preferably from 91/9 to 92/8. Within such ranges, the solubility in water or the molecular weight or molecular weight distribution control of the resulting polymer (A) are further improved.

In addition to the water-soluble vinyl monomer (a1) and the hydrolyzable vinyl monomer (a2), an additional vinyl monomer (a3) copolymerizable with the aforementioned vinyl monomers can be used as a raw material vinyl monomer. The additional vinyl monomer (a3) may be used singly or two or more of the same may be used in combination.

The additional copolymerizable vinyl monomer (a3) is not particularly limited and conventional hydrophobic vinyl monomers (e.g., hydrophobic vinyl monomers disclosed in paragraphs 0028 to 0029 of Japanese Patent No. 3648553, vinyl monomers disclosed in paragraph 0025 of JP-A-2003-165883 and paragraph 0058 of JP-A-2005-75982) can be used, and specifically, for example, the following vinyl monomers (i) to (iii) can be used.

(i) Aromatic ethylenic monomers having 8 to 30 carbon atoms Styrenes, such as styrene, α-methylstyrene, vinyltoluene, and hydroxystyrene, vinylnaphthalene, and halogenated forms of styrene, such as dichlorostyrene, etc.
(ii) Aliphatic ethylenic monomers having 2 to 20 carbon atoms
Alkenes (e.g., ethylene, propylene, butene, isobutylene, pentene, heptene, diisobutylene, octene, dodecene, and octadecene), and alkadienes (e.g., butadiene and isoprene), etc.
(iii) Alicyclic ethylenic monomers having 5 to 15 carbon atoms
Monoethylenically unsaturated monomers (e.g., pinene, limonene, and indene); and polyethylenic vinyl monomers (e.g., cyclopentadiene, bicyclopentadiene, and ethylidene norbornene), etc.

From the viewpoint of the solubility in water or the molecular weight or molecular weight distribution control of a polymer, in the raw material vinyl monomer, the content (mol%) of the additional vinyl monomer (a3), based on the total

number of moles of the water-soluble vinyl monomer (a1) and the hydrolyzable vinyl monomer (a2), is preferably 0 to 5, more preferably 0 to 3, even more preferably 0 to 2, and particularly preferably 0 to 1.5, and from the viewpoint of the solubility in water or the molecular weight or molecular weight distribution control of a polymer, the content of the additional vinyl monomer (a3) is most preferably 0 mol%.

In the production method of the present invention, the usage of the molecular weight controlling agent for radical polymerization in terms of the amount of the active ingredient is preferably 0.0005 to 1% by weight, and more preferably 0.005 to 0.5% by weight, based on the total weight of the raw material vinyl monomer, that is, the weight of the above-mentioned monomers (a1) and (a2), or when an additional monomer (a3) is also used, (a1) to (a3). When the amount of the molecular weight controlling agent for radical polymerization is less than 0.0005% by weight in the amount of the active ingredient, there is a possibility that polymerization cannot be controlled sufficiently, so that the molecular weight distribution of a resulting polymer will be broad. On the other hand, when it exceeds 1% by weight, there is a possibility that the molecular chain of a resulting polymer will be excessively short, and economic efficiency is poor.

In the method for producing a polymer of the present invention, radical polymerization may also be carried out in the presence of an internal crosslinking agent (b). By performing radical polymerization in the presence of an internal crosslinking agent (b) (hereinafter referred to as radical crosslinking polymerization), a crosslinked polymer represented by a water-absorbent resin can be synthesized. By using the molecular weight controlling agent for radical polymerization of the present invention in radical crosslinking polymerization, it is considered that the molecular weight between crosslinking points can be easily made uniform, and it is expected that the absorption performance and the mechanical properties will be improved as compared with conventional polymerization methods.

The internal crosslinking agent (b) is not particularly limited, and conventional crosslinking agents (e.g., crosslinking agents having two or more ethylenically unsaturated groups disclosed in paragraphs 0031 to 0034 of Japanese Patent No. 3648553, crosslinking agents having at least one functional group capable of reacting with a water-soluble substituent and having at least one ethylenically unsaturated group and crosslinking agents having at least two functional groups each capable of reacting a water-soluble substituent, crosslinking agents having two or more ethylenically unsaturated groups, crosslinking agents having an ethylenically unsaturated group and a reactive functional group and crosslinking agents having two or more reactive substituents disclosed in paragraphs 0028 to 0031 of JP-A-2003-165883, crosslinkable vinyl monomers disclosed in paragraph 0059 of JP-A-2005-75982 and crosslinkable vinyl monomers disclosed in paragraphs 0015 to 0016 of JP-A-2005-95759) can be used. Among these, from the viewpoint of absorption performance, etc., crosslinking agents having two or more ethylenically unsaturated groups are preferable, and more preferable are bis(meth)acrylamides such as N,N'-methylenebisacrylamide; poly(meth)acrylates of polyhydric alcohols such as (poly)alkylene glycol, trimethylolpropane, glycerin, pentaerythritol and sorbitol; poly(meth)allyl ethers of polyhydric alcohols such as (poly)alkylene glycols, trimethylolpropane, glycerin, pentaerythritol and sorbitol; and polyvalent (meth)allyl compounds such as tetraallyloxyethane and triallyl isocyanurate, and most preferred are polyvalent (meth)allyl compounds. The internal crosslinking agent (b) may be used singly or two or more of the same may be used in combination.

The content (mol%) of the internal crosslinking agent (b) units is preferably 0.001 to 5, more preferably 0.005 to 3, and particularly preferably 0.01 to 1 based on the total number of moles of the water-soluble vinyl monomer (a1) units and the hydrolyzable vinyl monomer (a2) units or, in the case where the additional vinyl monomer (a3) is used, the total number of moles of (a1) to (a3). Within such ranges, the molecular weight and the molecular weight distribution control of a polymer are further improved.

In the production method of the present invention, the step of polymerizing a raw material vinyl monomer comprising a water-soluble vinyl monomer (a1) and/or a hydrolyzable vinyl monomer (a2) in the presence of the above-described molecular weight controlling agent for radical polymerization and water may be performed by carrying out conventional polymerization such as aqueous solution polymerization (adiabatic polymerization, film polymerization, spray polymerization, etc.; e.g., JP-A-55-133413) and inverse phase suspension polymerization (e.g., JP-B-54-30710, JP-A-56-26909, and JP-A-1-5808) in the presence of the above-described molecular weight controlling agent for radical polymerization.

When performing aqueous solution polymerization, a mixed solvent comprising water and an organic solvent can be used. Examples of the organic solvent include methanol, ethanol, acetone, methyl ethyl ketone, N,N-dimethylformamide, dimethylsulfoxide, and mixtures of two or more thereof.

When performing aqueous solution polymerization, the usage (% by weight) of an organic solvent is preferably 40 or less, and more preferably 30 or less, based on the weight of water.

When the polymerization method is
an inverse phase suspension polymerization method, polymerization may be carried out in the presence of a conventional dispersing agent or a conventional surfactant, if necessary. In the case of an inverse phase suspension polymerization method, a conventional hydrocarbon solvent such as xylene, n-hexane, and n-heptane can be used in combination with water.

Among the polymerization methods, the aqueous solution polymerization method is preferred because it is

advantageous in production cost due to no need for use of an organic solvent.

In the polymerization, the weight percent concentration of the raw material vinyl monomer comprising the water-soluble vinyl monomer (a1) and/or the hydrolyzable vinyl monomer (a2), and when an additional vinyl monomer (a3) is used, (a1) to (a3), and the internal crosslinking agent (b) is preferably 20 to 55% relative to the total weight of the polymerization liquid at the initiation of the polymerization. When the concentration is lower than this range, the productivity is deteriorated, and when the concentration is higher, it is difficult to control the temperature in the system due to polymerization heat and it is difficult to industrially stably obtain a polymer having controlled molecular weight and molecular weight distribution.

In the polymerization of the raw material vinyl monomer described above, a conventional radical initiator can be used, if necessary. Examples of the conventional radical initiator include azo compounds [e.g., azobisisobutyronitrile, azobiscyanovaleric acid, 2,2'-azobis[N-(2-hydroxyethyl)-2-methylpropaneamide], and 2,2'-azobis(2-amidinopropane) hydrochoride], inorganic peroxides (e.g., hydrogen peroxide, ammonium persulfate, potassium persulfate, and sodium persulfate), organic peroxides [benzoyl peroxide, di-t-butyl peroxide, cumene hydroperoxide, succinic acid peroxide, and di(2-ethoxyethyl) peroxydicarbonate], redox catalysts (combinations of a reducing agent such as alkali metal sulfite or bisulfite, ammonium sulfite, ammonium bisulfite and ascorbic acid and an oxidizing agent such as alkali metal persulfates, ammonium persulfate, hydrogen peroxide, and an organic peroxides), and photoradical generators [e.g., 2,4,6-trimethylbenzoyl-diphenyl-phosphine oxide, 1-hydroxycyclohexyl-phenyl ketone-hydroxyalkylphenone, and $\alpha$-aminoalkylphenone]. Such radical initiators may be used singly and two or more thereof may be used in combination.

The usage (% by weight) of the radical initiator is preferably 0.0005 to 5, and more preferably 0.001 to 2, based on the total weight of the water-soluble vinyl monomer (a1) and the hydrolyzable vinyl monomer (a2), or when an additional vinyl monomer (a3) is used, (a1) to (a3).

The polymerization initiation temperature in polymerizing the raw material vinyl monomer comprising the water-soluble vinyl monomer (a1) and/or the hydrolyzable vinyl monomer (a2) in the presence of the above-described molecular weight controlling agent for radical polymerization is preferably 0 to 80°C. When the temperature is lower than this range, it is difficult to perform production because a polymerization liquid may be frozen, and when the temperature is higher, it is difficult to control the temperature in the system due to polymerization heat and it is difficult to industrially stably obtain a polymer having controlled molecular weight and molecular weight distribution.

The molecular weight distribution of a polymer to be obtained by the production method of the present invention is preferably 4 or less, more preferably 3.9 or less, and particularly preferably 3.8 or less. When the molecular weight distribution exceeds 4, the ingredients in the molecular weight range contributing to development of the desired performance will decrease, so the performance will be insufficient. The molecular weight distribution is measured by the method described later.

In the production method of the present invention, the mixture comprising the polymer may be subjected to secondary treatment such as concentration and drying as necessary.

When water and/or an organic solvent is distilled off from the mixture comprising the polymer, the content (% by weight) of water and/or the organic solvent after distillation, based on the weight of the polymer, is preferably 0 to 20, more preferably 0.5 to 10, particularly preferably 1 to 9, and most preferably 2 to 8. Within such ranges, the performance of the polymer is improved.

The contents of an organic solvent and water can be determined from the weight loss of a measurement sample when heating it with an infrared moisture content analyzer {e.g., JE400 manufactured by KETT; 120 $\pm$ 5°C, 30 minutes, atmosphere humidity before heating: 50 $\pm$ 10%RH, lamp specification: 100 V, 40 W}, but the determination is not limited to this.

In the production method of the present invention, when the radical crosslinking polymerization is carried out in the presence of an internal crosslinking agent (b), it is preferable that the hydrous gel containing a resulting polymer be chopped if necessary and then the water and/or the organic solvent be distilled off.

When the hydrous gel is chopped, the size (longest diameter) of the chopped gel is preferably 50 $\mu$m to 10 cm, more preferably 100 $\mu$m to 2 cm, and particularly preferably 1 mm to 1 cm. When the size is within such ranges, dryability during a drying step is further improved.

Chopping can be carried out by a conventional method and chopping can be done by using a chopping machine (e.g., Bex Mill, rubber chopper, Pharma Mill, mincing machine, impact type pulverizer, roll type pulverizer), etc. In addition, when using a vinyl monomer containing an acid group such as a carboxyl group, the hydrous gel of the polymer obtained as described above may be mixed with an alkali to be neutralized, if necessary.

As the alkali, known one (gazette of Japan Patent No. 3205168, etc.) can be used. Of these, lithium hydroxide, sodium hydroxide, and potassium hydroxide are preferred from the viewpoint of water absorption performance; more preferred are sodium hydroxide and potassium hydroxide, and particularly preferred is sodium hydroxide. From the viewpoint of liquid permeation property, the neutralization ratio is preferably 50 to 100%, and more preferably 60 to 80%.

The production method of the present invention can be suitably used for the production of water-absorbent resin particles. In this case, the production method preferably comprises a step of pulverizing the hydrous gel after distilling off

water and/or an organic solvent from the hydrous gel containing the water-absorbent resin, and water-absorbent resin particles are obtained through the pulverization. The method of pulverization is not particularly limited and pulverizing apparatuses (e.g., hammer type pulverizer, impact type pulverizer, roll type pulverizer, and jet stream type pulverizer) can be used. The water-absorbent resin particles obtained by the pulverization can be adjusted in their particle size by screening, etc., according to need.

The weight average particle diameter (μm) of the water-absorbent resin particles when having been screened according to need is preferably 100 to 800, more preferably 200 to 700, even more preferably 250 to 600, particularly preferably 300 to 500, and most preferably 350 to 450. Within such ranges, the absorption performance is further improved.

The weight average particle diameter of the water-absorbent resin particles is measured by the method disclosed in Perry's Chemical Engineers' Handbook, Sixth Edition (McGraw-Hill Book Company, 1984, page 21) by using a RO-TAP screen shaker and standard screens (JIS Z8801-1:2006). Specifically, JIS standard screens are combined, for example, in the order of 1000 μm, 850 μm, 710 μm, 500 μm, 425 μm, 355 μm, 250 μm, 150 μm, 125 μm, 75 μm, 45 μm, and a bottom tray when viewed from the top. About 50 g of particles to be measured are put on the top screen and then shaken for five minutes by a RO-TAP screen shaker. Then, the particles to be measured received on the respective screens and the bottom tray are weighed and the weight fractions of the particles on the respective screens are calculated with the total weight of the particles considered to be 100% by weight. The calculated values are plotted on a logarithmic probability sheet {taking the size of openings of a screen (particle diameter) as abscissa and the weight fraction as ordinate} and then a line connecting the respective points is drawn. Subsequently, a particle diameter that corresponds to a weight fraction of 50% by weight is determined and this is defined as a weight average particle diameter.

A smaller content of particulates contained in the water-absorbent resin particles results in better absorption performance, and the content (% by weight) of the particulates being 106 μm or less in size (preferably being 150 μm or less in size) that accounts for in the total weight of the water-absorbent resin particles is preferably 3 or less, and more preferably 1 or less. The content of the particulates can be determined using a graph produced when determining the aforementioned weight average particle diameter.

The shape of the water-absorbent resin particles after performing the step of pulverization is not particularly limited and may be an irregularly pulverized form, a scaly form, a pearl-like form, a rice grain form, etc. Among these, an irregularly pulverized form is preferred because good entangling with a fibrous material in an application such as disposable diaper is ensured and the fear of falling off from the fibrous material is eliminated.

In the case of producing water-absorbent resin particles by the production method of the present invention, the production method preferably further comprises a step of surface-crosslinking the water-absorbent resin. By surface-crosslinking, it is possible to further enhance gel strength and it is possible to satisfy a desirable water retaining capacity and an amount of absorption under load in practical use.

A method for surface-crosslinking the water-absorbent resin may be a conventional method, e.g., a method in which a water-absorbent resin is processed into a particle form, and then is mixed with a mixed solution of a surface-crosslinking agent (d), water and a solvent and then heating reaction is performed. A method of the mixing may be spraying the above-mentioned mixed solution to the water-absorbent resin, dipping the crosslinked polymer in the above-mentioned mixed solution, or the like, and preferred is a method of spraying the above-mentioned mixed solution to water-absorbent resin and then mixing them.

Examples of the surface-crosslinking agent (d) include polyglycidyl compounds, such as ethylene glycol diglycidyl ether, glycerol diglycidyl ether, and polyglycerol polyglycidyl ether; polyhydric alcohols, such as glycerol and ethylene glycol; ethylene carbonate, polyamines, and polyvalent metal compounds. Of these, preferred in that a crosslinking reaction can be performed at a relatively low temperature are the polyglycidyl compounds. Such surface-crosslinking agents may be used singly or two or more of them may be used in combination.

The usage of the surface-crosslinking agent (d) is preferably 0.001 to 5% by weight, and more preferably 0.005 to 2% by weight, based on the weight of the water-absorbent resin before crosslinking. When the amount of the surface-crosslinking agent (d) used is less than 0.001% by weight, there is a possibility that the degree of crosslinking of a surface is not high enough and the effect of increasing the amount of absorption under load is insufficient. On the other hand, when the usage of (d) exceeds 5% by weight, there is a possibility that the degree of crosslinking of a surface is excessively high and the water retaining capacity lowers.

The usage of the water during surface-crosslinking is preferably 1 to 10% by weight, and more preferably 2 to 7% by weight, based on the weight of the water-absorbent resin before crosslinking. When the usage of the water is less than 1% by weight, there is a possibility that the degree of permeation of the surface-crosslinking agent (d) into the inside of water-absorbent resin particles is not high enough and the effect of increasing the amount of absorption under load is insufficient. On the other hand, if the amount of the water used exceeds 10% by weight, there is a possibility that the permeation of the surface-crosslinking agent (d) into the inside is excessively high and the water retaining capacity lowers though increase in the amount of absorption under load is observed.

As a solvent to be used together with water for surface-crosslinking, a conventional solvent can be used through

appropriate selection taking into account the degree of permeation of the surface-crosslinking agent (d) into the water-absorbent resin particles, the reactivity of the surface-crosslinking agent (d), and preferably, the solvent is a hydrophilic organic solvent that is soluble in water, such as methanol and diethylene glycol. Such solvents may be used singly or two or more of them may be used in combination.

The usage of the solvent may be appropriately adjusted depending on the type of the solvent, and it is preferably 1 to 10% by weight based on the weight of the water-absorbent resin before surface-crosslinking. The ratio of the solvent to water may also be arbitrarily adjusted, and it is preferably 20 to 80% by weight, and more preferably 30 to 70% by weight.

In order to perform surface-crosslinking, the mixed solution of the surface-crosslinking agent (d), water and the solvent is mixed with water-absorbent resin particles, and then a heating reaction is performed. The reaction temperature is preferably 100 to 230°C, and more preferably 120 to 160°C. The reaction time can appropriately be adjusted depending on the reaction temperature, and it is preferably 3 to 60 minutes, and more preferably 10 to 40 minutes. The granular water-absorbent resin obtained by surface-crosslinking may further be surface-crosslinked using a surface-crosslinking agent of the same type or a different type from the surface-crosslinking agent used first.

In the production of the water-absorbent resin particles, a step of adjusting the particle size by screening may, if necessary, be carried out after the surface-crosslinking. The weight-average particle diameter of the particles obtained after the particle size adjustment is preferably 100 to 600 μm, and more preferably 200 to 500 μm. The content of particulates is preferred to be as small as possible; the content of particle being equal to or smaller than 100 μm is preferably 3% by weight or less, and it is more preferred that the content of particles being equal to or smaller than 150 μm be 3% by weight or less.

In the production of water-absorbent resin particles, antiseptics, antifungal agents, antibacterial agents, antioxidants, UV absorbers, coloring agents, aromatics, deodorants, liquid permeation property improvers, inorganic powders, organic fibrous materials, etc. may be added at an arbitrary stage, and the amount thereof is 5% by weight or less based on the weight of the water-absorbent resin obtained. Moreover, treatment to form a foamed structure may be performed at an arbitrary stage in the method of the present invention, if necessary, and granulation and molding may also be performed.

The water retaining capacity (g/g) with respect to physiological saline at 25°C of water-absorbent resin particles to be obtained by the production method of the present invention is preferably 30 or more, more preferably 32 or more, and particularly preferably 34 or more. When it is less than 30, the leakage property of an absorbent article becomes worse. The water retaining capacity is measured by the method described later.

The apparent density (g/ml) of the water-absorbent resin particles obtained by the production method of the present invention is preferably 0.54 to 0.70, more preferably 0.56 to 0.65, and particularly preferably 0.58 to 0.60. Within such ranges, the skin irritation resistance of an absorbent article is further improved. The apparent density can be measured at 25°C in accordance with JIS K7365:1999, for example.

The water-absorbent resin particles to be obtained by the production method of the present invention may be used alone as an absorbent body, or may be used as an absorber together with other materials.

Examples of the other materials include a fibrous material. The structure, the production method, etc. of the absorbent body in the case of using together with a fibrous material are analogous to conventional structures and methods (JP-A-2003-225565, JP-A-2006-131767, JP-A-2005-097569, etc.).

Preferred as the fibrous material are cellulosic fiber, organic synthetic fiber and mixtures of a cellulosic fiber and an organic synthetic fiber.

Examples of the cellulosic fiber include natural fibers such as fluff pulp and cellulosic chemical fibers such as viscose rayon, acetate rayon, and cuprammonium rayon. Such cellulosic natural fibers are not particularly limited with respect to their source material (needle-leaf trees, broadleaf trees, etc.), production method (chemical pulp, semichemical pulp, mechanical pulp, CTMP, etc.), bleaching method, etc.

Examples of the organic synthetic fiber include polypropylene-based fiber, polyethylene-based fiber, polyamide-based fiber, polyacrylonitrile-based fiber, polyester-based fiber, polyvinyl alcohol-based fiber, polyurethane-based fiber, and heat-weldable composite fiber (fiber in which at least two of the fibers differing in melting point are hybridized in a sheath-core type, an eccentric type, a parallel type, or the like, fiber in which at least two of the fibers are blended, and fiber in which the surface layer of the fibers is modified, etc.).

Preferred among these fibrous base materials are cellulosic natural fiber, polypropylene-based fiber, polyethylene-based fiber, polyester-based fiber, heat-weldable composite fiber, and mixed fiber thereof, and fluff pulp, heat-weldable fiber, and mixed fiber thereof are more preferred in that a resulting water absorber is excellent in shape retention after water absorption.

The fibrous material is not particularly limited in length and thickness, and it can suitably be used if its length is within a range of 1 to 200 mm and its thickness is within a range of 0.1 to 100 deniers. The shape thereof is also not particularly limited if it is fibrous, and examples of the shape include a narrow cylindrical form, a split yarn form, a staple form, a filament form, and a web form.

When the water-absorbent resin particles are processed together with a fibrous material to form an absorbent

body, the weight ratio of the water-absorbent resin particles to the fiber (the weight of the water-absorbent resin particles/the weight of the fiber) is preferably from 40/60 to 90/10, and more preferably from 70/30 to 80/20.

The absorbent body comprising the water-absorbent resin particles described above can be used as an absorbent article. The absorbent article can be applied not only as sanitary goods such as disposable diaper or sanitary napkin but also as items to be used for various applications such as absorbent materials or retention materials for various types of aqueous liquid, a gelling agent, etc. The method for producing the absorbent article is analogous to conventional methods (those disclosed in JP-A-2003-225565, JP-A-2006-131767, and JP-A-2005-097569, etc.).

EXAMPLES

The present invention is further described below by means of Examples and Comparative Examples, but the present invention is not limited thereto. Hereafter, unless otherwise stated, "part(s)" means "part(s) by weight" and "%" means "% by weight". The molecular weight and the molecular weight distribution of polymers, the water retaining capacity of water-absorbent resin particles with respect to physiological saline, the amount of absorption under load, and the gel liquid permeation rate were measured by the following methods.

<Method 1 for measuring molecular weight and molecular weight distribution>

The number average molecular weight (Mn), the weight average molecular weight (Mw), and the molecular weight distribution PDI (Mw/Mn) of polymers having no cationic group were measured using the following apparatus and conditions.

[1] Instrument: gel permeation chromatograph "HLC-8120GPC", manufactured by Tosoh Corporation.
[2] Column: "TSKgel G6000PWxl" and "TSKgel G3000PWxl" [both manufactured by Tosoh Corporation] are connected in series.
[3] Eluent: solution prepared by dissolving 0.5% by weight of sodium acetate in methanol/water = 30/70 (volume ratio).
[4] Reference substance: polyethylene glycol
[5] Injection conditions: sample concentration: 0.25% by weight, column temperature: 40°C.

<Method 2 for measuring molecular weight and molecular weight distribution>

The number average molecular weight (Mn), the weight average molecular weight (Mw), and the molecular weight distribution PDI (Mw/Mn) of polymers having a cationic group were measured using the following apparatus and conditions.

[1] Instrument: gel permeation chromatograph "HLC-8120GPC", manufactured by Tosoh Corporation.
[2] Column: "TSKgel G6000PWxl-CP" and "TSKgel G3000PWxl-CP" [both manufactured by Tosoh Corporation] are connected in series.
[3] Eluent: solution prepared by dissolving 0.5% by weight of sodium acetate in methanol/water = 30/70 (volume ratio).
[4] Reference substance: polyethylene glycol
[5] Injection conditions: sample concentration: 0.25% by weight, column temperature: 40°C.

<Method for measuring water retaining capacity>

1.00 g of a measurement sample was put into a tea bag (20 cm long, 10 cm wide) made of nylon net with a size of openings of 63 μm (JIS Z8801-1:2006) and then was immersed in 1,000 ml of physiological saline (salt concentration: 0.9%) for 1 hour without stirring, followed by pulling up and draining off water by hanging the sample for 15 minutes. Then, the sample in the tea bag was put in a centrifuge and centrifugally dewatered at 150 G for 90 seconds, thereby removing excess physiological saline. Subsequently, the weight (h1) of the sample including the tea bag was measured and then a water retaining capacity was calculated from the following formula. (h2) is the weight of the tea bag measured with no measurement sample by analogous procedures to those described above. The temperature of the physiological saline used and that of the measurement atmosphere were 25°C ± 2°C.

$$\text{Water retaining capacity (g/g)} = (h1) - (h2)$$

<Method for measuring the amount of absorption under load>

Into a cylindrical plastic tube (inner diameter: 25 mm, height: 34 mm) with a nylon net having a size of openings of 63 μm (JIS Z8801-1:2006) attached to the bottom of the tube, there was weighed 0.16 g of a measurement sample screened into a range of 250 to 500 μm using a 30 mesh screen and a 60 mesh screen, and then the cylindrical plastic tube was made to stand vertically and the measurement sample was leveled to have an almost uniform thickness on the nylon net and then a weight (weight: 310.6 g, outer diameter: 24.5 mm) was put on the measurement sample. The weight (M1) of the cylindrical plastic tube as the whole was measured, and then the cylindrical plastic tube containing the measurement sample and the weight was made to stand in a petri dish (diameter: 12 cm) containing 60 ml of physiological saline (salt concentration: 0.9%) and was immersed with the nylon net side facing down and was left standing for 60 minutes. After a lapse of 60 minutes, the cylindrical plastic tube was pulled up from the petri dish and then was inclined to concentrate the water attaching to the bottom of the tube to drip in the form of water drops, thereby removing excess water. Then, the weight (M2) of the cylindrical plastic tube containing the measurement sample and the weight as the whole was measured and then the amount of absorption under load was determined from the following formula. The temperature of the physiological saline used and that of the measurement atmosphere were 25°C ± 2°C.

The amount (g/g) of absorption under load = {(M2) - (M1) }/0.16

<Method for measuring gel liquid permeation rate>

The gel liquid permeation rate was measured by the following operations using the instruments illustrated in Figs. 1 and 2.

Swollen gel particles 2 are prepared by immersing 0.32 g of a measurement sample in 150 ml of physiological saline 1 (salt concentration: 0.9%) for 30 minutes. Then, into a filtration cylinder equipped with a wire gauze 6 (mesh size: 106 μm, JIS Z8801-1:2006) and a freely openable and closable shut-off cock 7 (inner diameter of the liquid permeation portion: 5 mm) at the bottom of a vertically standing cylinder 3 {diameter (inner diameter): 25.4 mm, length: 40 cm; there are graduation lines 4 and 5 at the positions of 60 ml and 40 ml from the bottom, respectively}, the prepared swollen gel particles 2 are transferred together with the physiological saline while closing the shut-off cock 7. Then, a pressing axis 9 (weight: 22 g, length: 47 cm) with a circular wire gauze 8 (size of openings: 150 μm, diameter: 25 mm) attached perpendicularly with respect to the wire gauze plane is put on the swollen gel particles 2 in such a manner that the wire gauze comes into contact with the swollen gel particles, and then a weight 10 (88.5 g) is put on the pressing axis 9 and is left standing for 1 minute. Subsequently, the cock 7 is opened and the time (T1; second) taken by the surface in the filtration cylinder to move from the 60 ml graduation line 4 to the 40 ml graduation line 5 is measured, and a gel liquid permeation rate (ml/min) is determined from the following formula.

Gel liquid permeation rate (ml/min) = 20 ml × 60/(T1 - T2)

The temperature of the physiological saline and that of the measurement atmosphere are 25°C ± 2°C, and T2 is the time measured by the same operation as described above for the case of using no measurement sample.

<Production of molecular weight controlling agent for radical polymerization>

(Reference Example 1) Production of 2-iodo-2-phenylacetic acid

8.4 parts of sodium iodide was added to a solution of 10.0 parts of 2-bromo-2-phenylacetic acid in 50 parts of acetone, followed by stirring at room temperature for 2 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 10.9 parts of 2-iodo-2-phenylacetic acid.
$^1$H NMR (400 MHz, $CDCl_3$): 55.56 (s, 1H), 7.33 (m, 3H), 7.61 (m, 2H)

(Reference Example 2) Production of 2-iodo-2-methylpropionitrile

50.7 parts of sodium iodide was added to a solution of 10.0 parts of 2-bromo-2-methylpropionitrile in 180 parts of acetone, followed by stirring at 55°C for 20 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 10.2 parts of 2-iodo-2-

methylpropionitrile.
$^1H$ NMR (400 MHz, $CDCl_3$): 52.22 (s, 6H)

(Reference Example 3) Production of α-iodobenzyl cyanide

9.2 parts of sodium iodide was added to a solution of 10.0 parts of α-bromobenzyl cyanide in 50 parts of acetone, followed by stirring at room temperature for 2 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 10.7 parts of α-iodobenzyl cyanide.
$^1H$ NMR (400 MHz, $CDCl_3$): δ5.62 (s, 1H), 7.38 (m, 3H), 7.54 (m, 2H)

(Reference Example 4) Production of ethyl 2-iodopropionate

9.9 parts of sodium iodide was added to a solution of 10.0 parts of ethyl 2-bromopropionate in 60 parts of acetone, followed by stirring at room temperature for 2 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 11.4 parts of ethyl 2-iodopropionate.
$^1H$ NMR (400 MHz, $CDCl_3$): δ1.28 (t, 3H), 1.96 (d, 3H), 4.21 (m, 2H), 4.47 (q, 1H)

(Reference Example 5) Production of ethyl 2-iodo-2-methylpropionate

38.4 parts of sodium iodide was added to a solution of 10.0 parts of ethyl 2-bromo-2-methylpropionate in 140 parts of acetone, followed by stirring at 55°C for 19 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 9.5 parts of ethyl 2-iodo-2-methylpropionate.
$^1H$ NMR (400 MHz, $CDCl_3$): δ1.30 (t, 3H), 2.08 (s, 6H), 4.22 (q, 2H)

The solubility in water at 20°C of each of the iodine compounds produced in Reference Examples 1 to 5 is as shown in Table 1.

[Table 1]

| | | Compound | Solubility in water (20°C) |
|---|---|---|---|
| Reference Examples | 1 | 2-Iodo-2-phenylacetic acid | 0.1% |
| | 2 | 2-Iodo-2-methylpropionitrile | 0.4% |
| | 3 | α-Iodobenzyl cyanide | <0.1% |
| | 4 | Ethyl 2-iodopropionate | 0.2% |
| | 5 | Ethyl 2-iodo-2-methylpropionate | <0.1% |

(Example 1) Production of 2-iodo-2-methylpropionic acid

44.9 parts of sodium iodide was added to a solution of 10.0 parts of 2-bromo-2-methylpropionic acid in 160 parts of acetone, followed by stirring at 55°C for 18 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 10.1 parts of 2-iodo-2-methylpropionic acid.
$^1H$ NMR (400 MHz, $CDCl_3$): δ2.10 (s, 6H)

(Example 2) Production of 2-iodopropionamide

11.8 parts of sodium iodide was added to a solution of 10.0 parts of 2-bromopropionic acid amide in 70 parts of acetone, followed by stirring at room temperature for 2 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 11.1

parts of 2-iodopropionamide.
$^1$H NMR (400 MHz, DMSO-$d_6$) : δ1. 75 (d, 3H), 4.47 (q, 1H), 7.27 (d, 2H, $NH_2$)

(Example 3) Preparation of 2-hydroxyethyl 2-iodo-2-methylpropionate

35.5 parts of sodium iodide was added to a solution of 10.0 parts of 2-hydroxyethyl 2-bromo-2-methylpropionate in 130 parts of acetone, followed by stirring at 55°C for 20 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 9.5 parts of 2-hydroxyethyl 2-iodo-2-methylpropionate.
$^1$H NMR (400 MHz, $CDCl_3$): δ2.06 (s, 6H), 2.42 (m, 1H, OH), 3.83 (m, 2H), 4.25 (m, 2H)

(Example 4) Production of α-iodo-γ-butyrolactone

10.9 parts of sodium iodide was added to a solution of 10.0 parts of α-bromo-γ-butyrolactone in 60 parts of acetone, followed by stirring at room temperature for 2 hours. Acetone in the reaction mixture was distilled off under reduced pressure and liquid separation extraction was performed using dichloromethane-water. The resulting organic layer was washed with saturated brine, dried over sodium sulfate, and distilled off under reduced pressure to obtain 11.2 parts of α-iodo-γ-butyrolactone.
$^1$H NMR (400 MHz, $CDCl_3$): δ2. 38 (m, 1H), 2.68 (m, 1H), 4.42 (m, 2H), 4.52 (m, 1H)

(Example 5) Production of sodium 2-iodo-2-methylpropionate

1.9 g of sodium hydroxide was added to a solution of 10.0 parts of 2-iodo-2-methylpropionic acid in 50 parts of ethanol, followed by stirring at room temperature for 30 minutes. The reaction mixture was distilled off under reduced pressure to obtain 10.9 parts of sodium 2-iodo-2-methylpropionate.
$^1$H NMR (400 MHz, $D_2O$): 51.92 (s, 6H)

(Example 6) Production of sodium 2-iodo-2-phenylacetate

1.5 parts of sodium hydroxide was added to a solution of 10.0 parts of 2-iodo-2-phenylacetic acid in 40 parts of ethanol, followed by stirring at room temperature for 30 minutes. The reaction mixture was distilled off under reduced pressure to obtain 10.7 parts of sodium 2-iodo-2-phenylacetate.
$^1$H NMR (400 MHz, $D_2O$): δ5.57 (s, 1H), 7.24 (m, 3H), 7.45 (m, 2H)
The solubility in water at 20°C of each of the iodine compounds produced in Examples 1 to 6 is as shown in Table 2.

[Table 2]

| | | Compound | Solubility in water (20°C) |
|---|---|---|---|
| Examples | 1 | 2-Iodo-2-methylpropionic acid | 1. 9% |
| | 2 | 2-Iodopropionamide | 1.2% |
| | 3 | 2-Hydroxyethyl 2-iodo-2-methylpropionate | 3.2% |
| | 4 | α-Iodo-γ-butyrolactone | 2.7% |
| | 5 | Sodium 2-iodo-2-methylpropionate | >10% |
| | 6 | Sodium 2-iodo-2-phenylacetate | >10% |

<Production of polymer>

(Example 7)

Under ice cooling, 172 parts of a 25% aqueous solution of sodium hydroxide was added to a mixed liquid of 105 parts of acrylic acid and 69 parts of ion-exchanged water with stirring and controlling temperature so as not to exceed 40°C, and thus neutralization was carried out. By introducing nitrogen gas into the solution, the amount of the oxygen dissolved in the solution was adjusted to less than or equal to 0.2 ppm and the solution temperature was adjusted to 10°C. To this

solution were added and mixed 1.7 parts of a 1% aqueous solution of 2-iodo-2-methylpropionic acid, 0.4 parts of a 1% aqueous solution of hydrogen peroxide, and 0.8 parts of a 2% aqueous solution of ascorbic acid, so that polymerization was started, and then a reaction was carried out for 6 hours, so that an aqueous solution containing a polymer (A-1) of the present invention was obtained. The Mn of the polymer (A-1) was 516,000, and the PDI was 3.35.

(Example 8)

An aqueous solution containing a polymer (A-2) of the present invention was obtained by performing the same operations as those of Example 7 except using 1.7 parts of a 1% aqueous solution of 2-iodopropionamide instead of 1.7 parts of the 1% aqueous solution of 2-iodo-2-methylpropionic acid in Example 7. The Mn of the polymer (A-2) was 602,000 and the PDI was 3.59.

(Example 9)

An aqueous solution containing a polymer (A-3) of the present invention was obtained by performing the same operations as those of Example 7 except using 1.7 parts of a 1% aqueous solution of 2-hydroxyethyl 2-iodo-2-methylpropionate instead of 1.7 parts of the 1% aqueous solution of 2-iodo-2-methylpropionic acid in Example 7. The Mn of the polymer (A-3) was 625,000, and the PDI was 3.44.

(Example 10)

An aqueous solution containing a polymer (A-4) of the present invention was obtained by performing the same operations as those of Example 7 except using 1.7 parts of a 1% aqueous solution of $\alpha$-iodo-$\gamma$-butyrolactone instead of 1.7 parts of the 1% aqueous solution of 2-iodo-2-methylpropionic acid in Example 7. The Mn of the polymer (A-4) was 588,000, and the PDI was 3.56.

(Example 11)

An aqueous solution containing a polymer (A-5) of the present invention was obtained by performing the same operations as those of Example 7 except using 1.7 parts of a 1% aqueous solution of sodium 2-iodo-2-methylpropionate instead of 1.7 parts of the 1% aqueous solution of 2-iodo-2-methylpropionic acid in Example 7. The Mn of the polymer (A-5) was 545,000, and the PDI was 3.36.

(Example 12)

An aqueous solution containing a polymer (A-6) of the present invention was obtained by performing the same operations as those of Example 7 except using 1.7 parts of a 1% aqueous solution of sodium 2-iodo-2-phenylacetate instead of 1.7 parts of the 1% aqueous solution of 2-iodo-2-methylpropionic acid in Example 7. The Mn of the polymer (A-6) was 637,000, and the PDI was 3.60.

(Example 13)

By introducing nitrogen gas into a mixed solution of 105 parts of acrylic acid and 242 parts of ion-exchanged water, the amount of the oxygen dissolved in the solution was adjusted to less than or equal to 0.2 ppm and the solution temperature was adjusted to 10°C. To this solution were added and mixed 1.7 parts of a 1% aqueous solution of 2-iodo-2-methylpropionic acid, 0.4 parts of a 1% aqueous solution of hydrogen peroxide, and 0.8 parts of a 2% aqueous solution of ascorbic acid, so that polymerization was started, followed by carrying out a reaction for 6 hours, and then 87 parts of a 49% aqueous solution of sodium hydroxide was added, followed by stirring for 24 hours and neutralization, so that an aqueous solution containing a polymer (A-7) of the present invention was obtained. The Mn of the polymer (A-7) was 646,000 and the PDI was 3.45.

(Example 14)

By introducing nitrogen gas into a mixed liquid of 100 parts of a 79% aqueous solution of methacryloyloxyethyltrimethylammonium chloride and 295 parts of ion-exchanged water, the amount of the oxygen dissolved in the solution was adjusted to less than or equal to 0.2 ppm and the solution temperature was adjusted to 45°C. To this solution were added and mixed 2.0 parts of a 5% aqueous solution of sodium 2-iodo-2-methylpropionate and 3.0 parts of a 5% aqueous solution of 2,2'-azobisamidinopropane dihydrochloride, so that polymerization was started, and then a reaction was

carried out for 6 hours, so that an aqueous solution containing a polymer (A-8) of the present invention was obtained. The Mn of the polymer (A-8) was 182,000 and the PDI was 1.61.

(Comparative Example 1)

An aqueous solution containing a comparative polymer (R-1) was obtained by performing the same operations as those of Example 7 except failing to use 1.7 parts of the 1% aqueous solution of 2-iodo-2-methylpropionic acid in Example 7. The Mn of the comparative polymer (R-1) was 397,000 and the PDI was 6.09.

(Comparative Example 2)

An aqueous solution containing a comparative polymer (R-2) was obtained by performing the same operations as those of Example 7 except using 0.02 parts of 2-iodo-2-phenylacetic acid instead of 1.7 parts of the 1% aqueous solution of 2-iodo-2-methylpropionic acid in Example 7. The Mn of the comparative polymer (R-2) was 412,000 and the PDI was 5.81.

(Comparative Example 3)

An aqueous solution containing a comparative polymer (R-3) was obtained by performing the same operations as those of Example 7 except using 0.02 parts of 2-iodo-2-methylpropionitrile instead of 1.7 parts of the 1% aqueous solution of 2-iodo-2-methylpropionic acid in Example 7. The Mn of the comparative polymer (R-3) was 472,000 and the PDI was 4.51.

(Comparative Example 4)

An aqueous solution containing a comparative polymer (R-4) was obtained by performing the same operations as those of Example 7 except using 0.02 parts of α-iodobenzyl cyanide instead of 1.7 parts of the 1% aqueous solution of 2-iodo-2-methylpropionic acid in Example 7. The Mn of the comparative polymer (R-4) was 407,000 and the PDI was 5.90.

(Comparative Example 5)

An aqueous solution containing a comparative polymer (R-5) was obtained by performing the same operations as those of Example 7 except using 0.02 parts of ethyl 2-iodopropionate instead of 1.7 parts of the 1% aqueous solution of 2-iodo-2-methylpropionic acid in Example 7. The Mn of the comparative polymer (R-5) was 421,000 and the PDI was 5.54.

(Comparative Example 6)

An aqueous solution containing a comparative polymer (R-6) was obtained by performing the same operations as those of Example 7 except using 0.02 parts of ethyl 2-iodo-2-methylpropionate instead of 1.7 parts of the 1% aqueous solution of 2-iodo-2-methylpropionic acid in Example 7. The Mn of the comparative polymer (R-6) was 427,000 and the PDI was 5.28.

(Comparative Example 7)

An aqueous solution containing a comparative polymer (R-7) was obtained by performing the same operations as those of Example 13 except using 0.02 parts of ethyl 2-iodopropionate instead of 1.7 parts of the 1% aqueous solution of 2-iodo-2-methylpropionic acid in Example 13. The Mn of the comparative polymer aqueous solution (R-7) was 746,000 and the PDI was 5.52.

(Comparative Example 8)

An aqueous solution containing a comparative polymer (R-8) was obtained by performing the same operations as those of Example 14 except using 0.1 parts of ethyl 2-iodopropionate instead of 2.0 parts of the 5% aqueous solution of 2-iodo-2-methylpropionic acid in Example 14. The Mn of the comparative polymer aqueous solution (R-8) was 226,000 and the PDI was 3.12.

The Mn and PDI of the resulting polymers (A-1) to (A-8) and the comparative polymers (R-1) to (R-8) are shown in Table 3.

[Table 3]

| | | Polymer | Monomer | Molecular weight controlli ng agent | Mn | PDI |
|---|---|---|---|---|---|---|
| Examples | 7 | (A-1) | Sodium acrylate | Example 1 | 516,000 | 3.35 |
| | 8 | (A-2) | Sodium acrylate | Example 2 | 602,000 | 3.59 |
| | 9 | (A-3) | Sodium acrylate | Example 3 | 625,000 | 3.44 |
| | 10 | (A-4) | Sodium acrylate | Example 4 | 588,000 | 3.56 |
| | 11 | (A-5) | Sodium acrylate | Example 5 | 545,000 | 3.36 |
| | 12 | (A-6) | Sodium acrylate | Example 6 | 637, 000 | 3.60 |
| | 13 | (A-7) | Acrylic acid | Example 1 | 646,000 | 3.45 |
| | 14 | (A-8) | Methacryloyloxyethyltrimethylammonium chloride | Example 5 | 182,000 | 1.61 |
| Com-parative Exam-ples | 1 | Comparative polymer (R-1) | Sodium acrylate | None | 397,000 | 6.09 |
| | 2 | Comparative polymer (R-2) | Sodium acrylate | Reference Example 1 | 412,000 | 5.81 |
| | 3 | Comparative polymer (R-3) | Sodium acrylate | Reference Example 2 | 472,000 | 4.51 |
| | 4 | Comparative polymer (R-4) | Sodium acrylate | Reference Example 3 | 407, 000 | 5.90 |
| | 5 | Comparative polymer (R-5) | Sodium acrylate | Reference Example 4 | 421,000 | 5.54 |
| | 6 | Comparative polymer (R-6) | Sodium acrylate | Reference Example 5 | 427,000 | 5.28 |
| | 7 | Comparative polymer (R-7) | Acrylic acid | Reference Example 4 | 746,000 | 5.52 |
| | 8 | Comparative polymer (R-8) | Methacryloyloxyethyltrimethylammonium chloride | Reference Example 4 | 226,000 | 3.12 |

From the results in Table 3, it can be seen that polymers of the present invention to be obtained using the molecular weight controlling agent for radical polymerization of the present invention are smaller in PDI and smaller in molecular weight distribution as compared with the polymers of the Comparative Examples.

<Production of water-absorbent resin particle>

(Example 15)

Under ice cooling, 247 parts of a 49% aqueous solution of sodium hydroxide was added to a mixed liquid of 300 parts of acrylic acid, polyethylene glycol #400 diacrylate (manufactured by Shin Nakamura Chemical Co., Ltd.) as a crosslinking agent, and 439 parts of ion-exchanged water with stirring and controlling temperature so as not to exceed 40°C, and thus neutralization was carried out to prepare an aqueous monomer solution. Then, the mixed liquid was charged into a polymerization vessel in which adiabatic polymerization can be performed. By introducing nitrogen gas into the solution, the amount of the oxygen dissolved in the solution was adjusted to less than or equal to 0.2 ppm and the solution temperature was adjusted to 10°C. To this polymerization solution were added and mixed 4.8 parts of a 1% aqueous solution of 2-iodo-2-methylpropionic acid, 1.2 parts of a 1% aqueous solution of hydrogen peroxide, 2.3 parts of a 2% aqueous solution of ascorbic acid, and 4.5 parts of a 2% aqueous solution of 2,2'-azobis[N-(2-hydroxyethyl)-2-methylpropanamide. At about 1 hour after the confirmation of the rise in temperature indicating the initiation of polymerization, the temperature arrived substantially at equilibrium at 90°C, and then aging was performed for additional

5 hours, affording a hydrous gel-like polymer.

The hydrous gel was chopped into small pieces of 5 to 10 mm by using scissors and further broken into small pieces by using a meat chopper, and then air-dried to have a water content of 4% under conditions including a supplied wind temperature of 150°C and a wind speed of 1.5 m/sec using a through hot air dryer (manufactured by Inoue Kinzoku Kogyo Co., Ltd.). The dried material was pulverized with a juicing blender (OSTERIZER BLENDER manufactured by Oster) and then screened to adjust it into a particle diameter range of from 710 to 150 μm expressed by size of openings, there by obtaining a water-absorbent resin particle (A1-1).

While stirring 100 parts of the water-absorbent resin (A1-1) (by using a high-speed stirring turbulizer manufactured by Hosokawa Micron Co.; rotation speed: 2000 rpm), a solution composed of 0.10 parts of ethylene glycol diglycidyl ether, 2.3 parts of water, 1.4 parts of propylene glycol, and 0.24 parts of sodium alum was added and mixed, and then surface-crosslinking was carried out by heating at 140°C for 45 minutes, and thus a water-absorbent resin particle (A2-1) of the present invention was obtained.

(Example 16)

A water-absorbent resin particle (A2-2) of the present invention was obtained by performing the same operations as those of Example 15 except changing the quantity of the 1% aqueous solution of 2-iodo-2-methylpropionic acid from 4.8 parts to 1.5 parts in Example 15.

(Example 17)

By mixing 300 parts of acrylic acid, 0.98 parts of pentaerythritol triallyl ether as a crosslinking agent (produced by OSAKA SODA CO., LTD.), and 687 parts of ion-exchanged water, an aqueous solution of the monomers was prepared, and then the mixed solution was supplied to a polymerization vessel in which adiabatic polymerization can be performed. By introducing nitrogen gas into the solution, the amount of the oxygen dissolved in the solution was adjusted to less than or equal to 0.2 ppm and the solution temperature was adjusted to 5°C. To this polymerization solution were added and mixed 4.8 parts of a 1% aqueous solution of 2-iodo-2-methylpropionic acid, 1.2 parts of a 1% aqueous solution of hydrogen peroxide, 2.3 parts of a 2% aqueous solution of ascorbic acid, and 4.5 parts of a 2% aqueous solution of 2,2'-azobis[N-(2-hydroxyethyl)-2-methylpropanamide. At about 1 hour after the confirmation of the rise in temperature indicating the initiation of polymerization, the temperature arrived substantially at equilibrium at 90°C, and then aging was performed for additional 5 hours, affording a hydrous gel-like polymer.

While chopping the hydrous gel-like polymer into chips using a meat chopper, 247 parts of a 49% aqueous solution of NaOH was added, converting about 72 mol% of the carboxyl groups in the polymer into sodium salt. The neutralized hydrous gel was through-dried to a water content of 4% under conditions including a supplied wind temperature of 150°C and a wind speed of 1.5 m/sec using a through hot air dryer (manufactured by Inoue Kinzoku Kogyo Co., Ltd.). The dried material was pulverized with a juicing blender (OSTERIZER BLENDER manufactured by Oster) and then screened to adjust it into a particle diameter range of from 710 to 150 μm expressed by size of openings, thereby obtaining a water-absorbent resin (A1-3).

While stirring 100 parts of the water-absorbent resin (A1-3) (by using a high-speed stirring turbulizer manufactured by Hosokawa Micron Co.; rotation speed: 2000 rpm), a solution composed of 0.10 parts of ethylene glycol diglycidyl ether, 2.3 parts of water, 1.4 parts of propylene glycol, and 0.24 parts of sodium alum was added and mixed, and then surface-crosslinking was carried out by heating at 140°C for 45 minutes, and thus a water-absorbent resin particle (A2-3) of the present invention was obtained.

(Example 18)

A water-absorbent resin particle (A2-4) of the present invention was obtained by performing the same operations as those of Example 17 except changing the quantity of the 1% aqueous solution of 2-iodo-2-methylpropionic acid from 4.8 parts to 1.5 parts in Example 17.

(Comparative Example 9)

A comparative water-absorbent resin particle (R2-1) was obtained by performing the same operations as those of Example 15 except failing to use 4.8 parts of the 1% aqueous solution of 2-iodo-2-methylpropionic acid in Example 15.

(Comparative Example 10)

A comparative water-absorbent resin particle (R2-2) was obtained by performing the same operations as those of

Example 15 except using 0.05 parts of ethyl 2-iodopropionate instead of 4.8 parts of the 1% aqueous solution of 2-iodo-2-methylpropionic acid in Example 15.

The evaluation results of water retaining capacity, amount of absorption under load, and gel liquid permeation rate of the obtained water-absorbent resin particles (A2-1) to (A2-4) and the comparative water-absorbent resin particles (R2-1) to (R2-2) for comparison were shown in Table 4.

[Table 4]

| | | Water-absorbent resin | Molecular weight controlling agent | Water retaining capacity (g/g) | Amount of absorption under load (g/g) | Gel liquid permeation rate (ml/min) |
|---|---|---|---|---|---|---|
| Examples | 15 | (A2-1) | Example 1 | 34 | 18 | 60 |
| | 16 | (A2-2) | Example 1 | 31 | 19 | 81 |
| | 17 | (A2-3) | Example 1 | 38 | 18 | 65 |
| | 18 | (A2-4) | Example 1 | 36 | 19 | 74 |
| Comparative Examples | 9 | Comparative water-absorbent resin (R2-1) | None | 27 | 17 | 63 |
| | 10 | Comparative water-absorbent resin (R2-2) | Reference Example 4 | 28 | 19 | 79 |

From the results in Table 4, it can be seen that water-absorbent resin particles to be obtained using the molecular weight controlling agent for radical polymerization of the present invention are higher in water retaining capacity as compared with the water-absorbent resin particles of the Comparative Examples. In particular, it can be seen that as compared with the water-absorbent resins of the Comparative Examples, the water-absorbent resins of the present invention were maintained in the amount of absorption under load and gel liquid permeation property at the same level while having superior water retaining capacity and thus the absorption performance were far advanced.

INDUSTRIAL APPLICABILITY

The molecular weight controlling agent for radical polymerization **disclosed herein** exhibits superior molecular weight control function in polymerization in the presence of water, particularly in an aqueous solution polymerization system, and is easy to handle, so that it can be used suitably for controlled radical polymerization of various water-soluble monomers. Moreover, polymers of water-soluble monomers obtained using the molecular weight controlling agent for radical polymerization **according to the present** invention have narrower molecular weight distribution and exhibit superior functions, such as superior viscosity increase, rheology control, dispersion, aggregation, and adhesion. Therefore, they are useful in the fields of cosmetics and toiletries, fiber processing, paints and inks, electronics, papermaking, civil engineering and construction, water treatment, and so on.

Water-absorbent resin particles to be obtained by the production method of the present invention can be compatible with the water retaining capacity, the liquid permeation property between swollen gel particles and the absorption performance under load and they can be processed by applying them to various types of absorbent bodies to form absorbent articles having a large amount of absorption and being superior in rewet performance or surface dry feeling. Accordingly, they are suitably used for sanitary goods, such as disposable diapers (disposable diaper for children, disposable diaper for adults, etc.), napkins (sanitary napkin, etc.), paper towel, pads (incontinence pad, surgical underpad, etc.), and pet sheets (pet urine absorbing sheet), and are best suited for disposable diapers. Moreover, water-absorbent resin particles to be obtained by the production method of the present invention are useful not only for sanitary goods but also for other various applications such as a pet urine absorbent, a urine gelling agent of a portable toilet, an agent for preserving freshness of vegetables and fruits etc., a drip absorbent for meats and fishes, a refrigerant, a disposable body warmer, a battery gelatinizer, a water retention agent for plants, soil, etc., a condensation preventing agent, a waterproofing agent, a packing agent, artificial snow, etc.

DESCRIPTION OF REFERENCE SIGNS

1 Physiological saline
2 Hydrous gel particles
3 Cylinder
4 Graduation line at the position of 60 ml from the bottom
5 Graduation line at the position of 40 ml from the bottom
6 Wire gauze
7 Shut-off cock
8 Circular wire gauze
9 Pressing axis
10 Weight

## Claims

1. A method for producing a polymer, comprising a step of carrying out radical polymerization of a raw material vinyl monomer comprising a water-soluble vinyl monomer (a1) and/or a vinyl monomer (a2) that becomes the water-soluble vinyl monomer (a1) through hydrolysis, wherein the radical polymerization is carried out in the presence of water and a molecular weight controlling agent for radical polymerization comprising an iodine compound represented by the following formula (1) as an active ingredient and having a solubility of the active ingredient in water of 0.5% by weight or more at 20°C,

   wherein the water-soluble vinyl monomer (a1) is a vinyl monomer soluble in an amount of at least 100 g in 100 g of water at 25°C,and wherein the radical polymerization of the raw material vinyl monomer is performed by carrying out aqueous solution polymerization or inverse phase suspension polymerization,

   [Chemical Formula 2]

   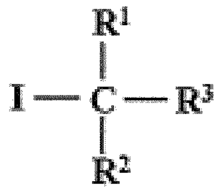

   (1)

   wherein $R^1$ is -COOX, -$CONR^4R^5$, an aromatic group or a cyano group, X is a hydrogen atom, an aliphatic group, an alkali metal, an alkaline earth metal, an organic ammonium or an ammonium, and $R^2$, $R^3$, $R^4$ and $R^5$ each independently represent a hydrogen atom, an aromatic group or an aliphatic group.

2. The method for producing a polymer according to claim 1, wherein two or more groups of $R^2$, $R^3$, $R^4$, $R^5$ and X are bonded to each other and form a saturated or unsaturated, 5- or 6-membered ring.

3. The method for producing a polymer according to claim 1 or 2, wherein the active ingredient has a carboxyl group, a salt of a carboxyl group, a hydroxyl group, or an amide group.

4. The method for producing a polymer according to any one of claims 1 to 3, wherein the solubility of the active ingredient of the molecular weight controlling agent for radical polymerization in water is 1% by weight or more at 20°C.

5. The method for producing a polymer according to any one of claims 1 to 4, wherein use of the molecular weight controlling agent for radical polymerization in terms of an amount of the active ingredient is 0.0005 to 1% by weight relative to a total weight of the raw material vinyl monomer.

6. The method for producing a polymer according to any one of claims 1 to 5, wherein the water-soluble vinyl monomer (a1) is (meth)acrylic acid (salt).

7. The method for producing a polymer according to any one of claims 1 to 6, wherein the radical polymerization is carried out in presence of an internal crosslinking agent (b).

8. The method for producing a polymer according to any one of claims 1 to 7, wherein a resulting polymer is a water-absorbent resin particle having a water retaining capacity of 30 g/g or more with respect to physiological saline at 25°C.

**Patentansprüche**

1. Ein Verfahren zur Herstellung eines Polymers, umfassend einen Schritt des Ausführens einer radikalischen Polymerisation eines Rohmaterial-Vinylmonomers, welches ein wasserlösliches Vinylmonomer (a1) und/oder ein Vinylmonomer (a2), das durch Hydrolyse zu dem wasserlöslichen Vinylmonomer (a1) wird, umfasst, wobei die radikalische Polymerisation in Gegenwart von Wasser und einem molekulargewichtssteuernden Mittel zur radikalischen Polymerisation, umfassend eine Iodverbindung, dargestellt durch die nachstehende Formel (1), als einen Wirkstoff und einer Löslichkeit des Wirkstoffes in Wasser von 0,5 Gew.-% oder mehr bei 20°C, ausgeführt wird,

   wobei das wasserlösliche Vinylmonomer (a1) ein Vinylmonomer ist, welches in einer Menge von mindestens 100 g in 100 g Wasser bei 25°C löslich ist, und wobei die radikalische Polymerisation des Rohmaterial-Vinylmonomers durch Ausführen einer Polymerisation in wässriger Lösung oder Inversphasen-Suspensionspolymerisation durchgeführt wird,

   [Chemische Formel 2]

   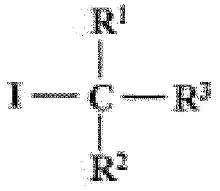

   (1)

   wobei $R^1$ für -COOX, -$CONR^4R^5$, eine aromatische Gruppe oder eine Cyanogruppe steht, X für ein Wasserstoffatom, eine aliphatische Gruppe, ein Alkalimetall, ein Erdalkalimetall, ein organisches Ammonium oder ein Ammonium steht, und $R^2$, $R^3$, $R^4$ und $R^5$ jeweils unabhängig ein Wasserstoffatom, eine aromatische Gruppe oder eine aliphatische Gruppe darstellen.

2. Das Verfahren zur Herstellung eines Polymers gemäß Anspruch 1, wobei zwei oder mehr Gruppen von $R^2$, $R^3$, $R^4$, $R^5$ und X miteinander verbunden sind und einen gesättigten oder ungesättigten 5- oder 6-gliedrigen Ring bilden.

3. Das Verfahren zur Herstellung eines Polymers gemäß Anspruch 1 oder 2, wobei der Wirkstoff eine Carboxylgruppe, ein Salz einer Carboxylgruppe, eine Hydroxylgruppe oder eine Amidgruppe aufweist.

4. Das Verfahren zur Herstellung eines Polymers gemäß einem der Ansprüche 1 bis 3, wobei die Löslichkeit des Wirkstoffes des molekulargewichtssteuernden Mittels für radikalische Polymerisation in Wasser 1 Gew.-% oder mehr bei 20°C beträgt.

5. Das Verfahren zur Herstellung eines Polymers gemäß einem der Ansprüche 1 bis 4, wobei die Verwendung des molekulargewichtssteuernden Mittels für radikalische Polymerisation, in Form einer Menge des Wirkstoffes, 0,0005 bis 1 Gew.-%, bezogen auf ein Gesamtgewicht des Rohmaterial-Vinylmonomers, beträgt.

6. Das Verfahren zur Herstellung eines Polymers gemäß einem der Ansprüche 1 bis 5, wobei das wasserlösliche Vinylmonomer (a1) (Meth)acrylsäure(salz) ist.

7. Das Verfahren zur Herstellung eines Polymers gemäß einem der Ansprüche 1 bis 6, wobei die radikalische Polymerisation in Gegenwart eines inneren Vernetzungsmittels (b) durchgeführt wird.

8. Das Verfahren zur Herstellung eines Polymers gemäß einem der Ansprüche 1 bis 7, wobei ein resultierendes Polymer

ein wasserabsorbierendes Harzteilchen mit einem Wasserrückhaltevermögen von 30 g/g oder mehr, bezogen auf physiologische Kochsalzlösung bei 25°C, ist.

## Revendications

**1.** Méthode pour produire un polymère, comprenant une étape de mise en œuvre d'une polymérisation radicalaire d'une matière première monomère vinylique comprenant un monomère vinylique soluble dans l'eau (a1) et/ou un monomère vinylique (a2) qui devient le monomère vinylique soluble dans l'eau (a1) par hydrolyse, dans laquelle la polymérisation radicalaire est mise en œuvre en présence d'eau et d'un agent de régulation de la masse moléculaire pour la polymérisation radicalaire comprenant un composé iodé représenté par la formule (1) suivante en tant qu'ingrédient actif et ayant une solubilité de l'ingrédient actif dans l'eau de 0,5 % en poids ou plus à 20 °C,

dans laquelle le monomère vinylique soluble dans l'eau (a1) est un monomère vinylique soluble en une quantité d'au moins 100 g dans 100 g d'eau à 25 °C, et dans laquelle la polymérisation radicalaire de la matière première monomère vinylique est effectuée par mise en œuvre d'une polymérisation en solution aqueuse ou d'une polymérisation en suspension en phase inverse,

[formule chimique 2]

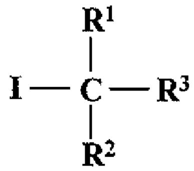 (1)

dans laquelle $R^1$ est -COOX, -$CONR^4R^5$, un groupe aromatique ou un groupe cyano, X est un atome d'hydrogène, un groupe aliphatique, un métal alcalin, un métal alcalino-terreux, un ammonium organique ou un ammonium, et chacun de $R^2$, $R^3$, $R^4$ et $R^5$ représente indépendamment un atome d'hydrogène, un groupe aromatique ou un groupe aliphatique.

**2.** Méthode pour produire un polymère selon la revendication 1, dans laquelle deux ou plus de deux groupes parmi $R^2$, $R^3$, $R^4$, $R^5$ et X sont liés entre eux et forment un cycle saturé ou insaturé à 5 ou 6 chaînons.

**3.** Méthode pour produire un polymère selon la revendication 1 ou 2, dans laquelle l'ingrédient actif a un groupe carboxyle, un sel d'un groupe carboxyle, un groupe hydroxyle, ou un groupe amide.

**4.** Méthode pour produire un polymère selon l'une quelconque des revendications 1 à 3, dans laquelle la solubilité dans l'eau de l'ingrédient actif de l'agent de régulation de la masse moléculaire pour la polymérisation radicalaire est de 1 % en poids ou plus à 20 °C.

**5.** Méthode pour produire un polymère selon l'une quelconque des revendications 1 à 4, dans laquelle l'utilisation de l'agent de régulation de la masse moléculaire pour la polymérisation radicalaire en termes de quantité de l'ingrédient actif est de 0,0005 à 1 % en poids par rapport au poids total de la matière première monomère vinylique.

**6.** Méthode pour produire un polymère selon l'une quelconque des revendications 1 à 5, dans laquelle le monomère vinylique soluble dans l'eau (a1) est un (sel de) acide (méth)acrylique.

**7.** Méthode pour produire un polymère selon l'une quelconque des revendications 1 à 6, dans laquelle la polymérisation radicalaire est mise en œuvre en présence d'un agent de réticulation interne (b).

**8.** Méthode pour produire un polymère selon l'une quelconque des revendications 1 à 7, dans laquelle le polymère résultant est une particule de résine absorbant l'eau ayant une capacité de rétention d'eau de 30 g/g ou plus par rapport à une solution salée physiologique à 25 °C.

Fig. 1

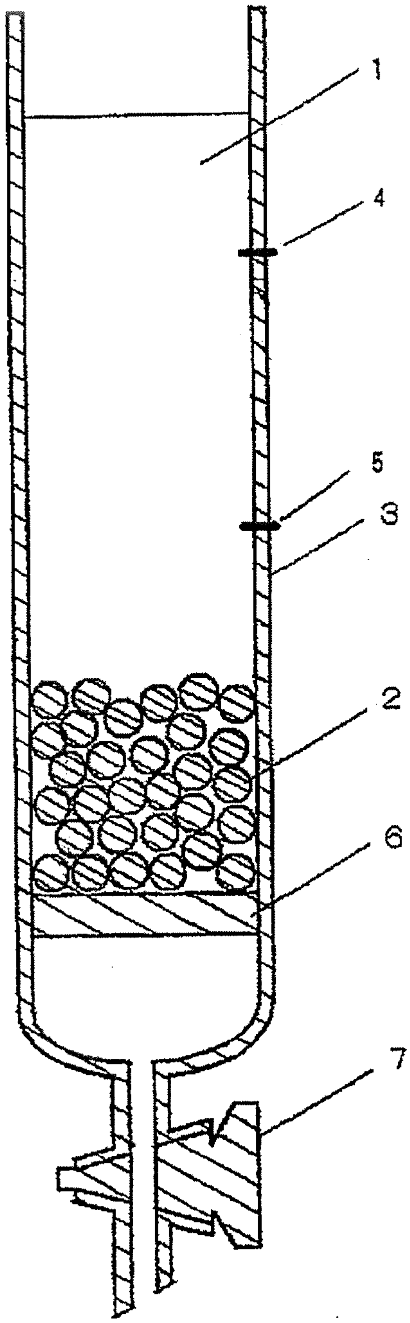

Fig. 2

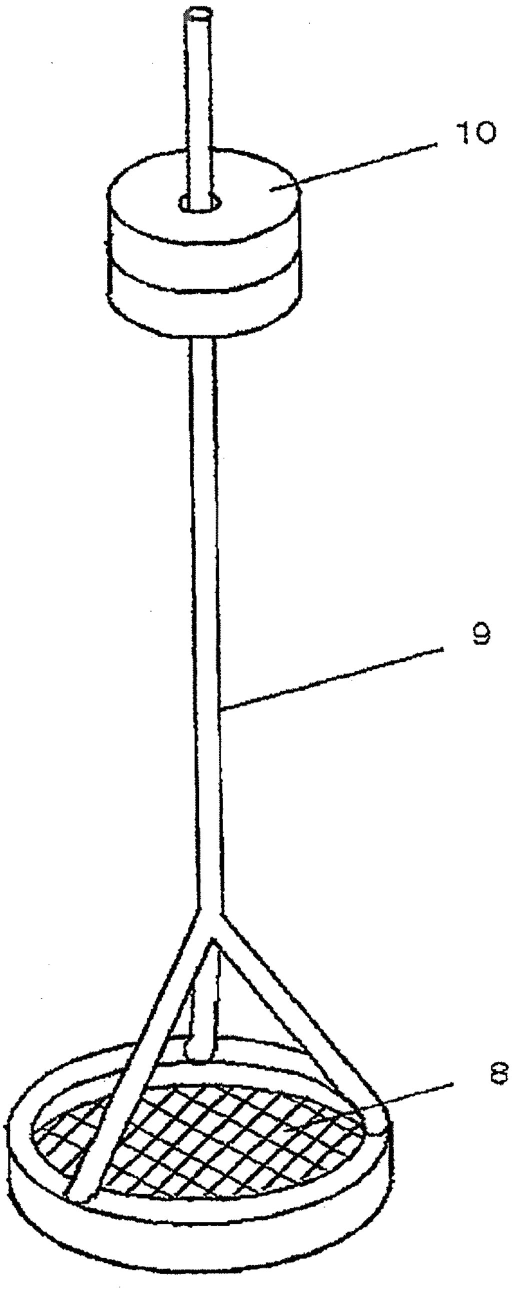

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11158206 A
- JP 2000515181 A
- JP 2006299278 A
- JP H11140127 A
- WO 2017057706 A1
- JP 62064814 A
- JP 2012062449 A
- JP 3648553 B
- JP 2003 A
- JP 165883 A
- JP 2005075982 A
- JP 2003165883 A
- JP 2005095759 A
- JP 55133413 A
- JP 54030710 B
- JP 56026909 A
- JP 1005808 A
- JP 3205168 B
- JP 2003225565 A
- JP 2006131767 A
- JP 2005 A
- JP 097569 A
- JP 2005097569 A


### Non-patent literature cited in the description


- Perry's Chemical Engineers' Handbook. McGraw-Hill Book Company, 1984, 21